(19) Europäisches Patentamt
European Patent Office
Office européen des brevets

(11) EP 1 716 852 A1

(12) EUROPEAN PATENT APPLICATION

(43) Date of publication:
**02.11.2006 Bulletin 2006/44**

(21) Application number: **06114107.3**

(22) Date of filing: **14.03.2001**

(51) Int Cl.:
***A61K 31/47*** *(2006.01)* ***A61K 31/675*** *(2006.01)*
***A61P 35/00*** *(2006.01)* *A61K 31/195* *(2006.01)*
*A61K 31/415* *(2006.01)*

(84) Designated Contracting States:
**AT BE CH CY DE DK ES FI FR GB GR IE IT LI LU MC NL PT SE TR**
Designated Extension States:
**AL LT LV MK RO SI**

(30) Priority: **17.03.2000 US 190007**

(62) Document number(s) of the earlier application(s) in accordance with Art. 76 EPC:
**01925510.8 / 1 267 873**

(71) Applicant: **Pfizer Enterprises SARL**
**1855 Luxembourg (LU)**

(72) Inventor: **BISSERY, Marie-Christine**
**94400, VITRY SUR SEINE (FR)**

(74) Representative: **Kendrick, David Alan**
**Pfizer Limited**
**European Patent Department**
**(IPC 748)**
**Ramsgate Road**
**GB-Sandwich, Kent CT13 9NJ (GB)**

Remarks:
This application was filed on 17-05- 2006 as a divisional application to the application mentioned under INID code 62.

## (54) A composition comprising campothecin or a campothecin derivative and an alkylating agent for the treatment of cancer

(57) The present invention relates to therapeutic associations for the treatment of cancer, comprising an effective amount of a campothecin, or a campothecin derivative, with an effective amount of an alkylating agent, such as melphalan, dacarbazine, or cyclophosphamide, and methods of using such therapeutic associations.

EP 1 716 852 A1

## Description

The present application claims the benefit of U.S. Provisional Application No. 60/190,007, filed March 17, 2000.

The present invention relates to therapeutic combinations comprising an effective amount of camptothecin, or a camptothecin derivative such as irinotecan (CPT-11), with an effective amount of an alkylating agent for the treatment of cancer.

More specifically, the invention relates to anticancer treatments with associations of camptothecin derivatives such as irinotecan (CPT-11, CAMPTOSAR®), topotecan, 9-aminocamptothecin, or 9-nitrocamptothecin, and alkylating agents. Such alkylating agents include, *inter alia,* melphalan (alkeran, L-3-{para-[Bis(2-chloroethyl)amino] phenyl} alanine, CB 3025, phenylalanine mustard, L-Sarcolysine, SK-15673), dacarbazine (DTIC-Dome®, DTIC, dimethyl triazeno imidazole carboxamide ; 5-3,3-dimethyl-l-triazenyl)-1H-imidazole-4-carboxamide, 9CI, DIC ), and cyclophosphamide (CPA, CYTOXAN®, NEOSAR®).

European patent EP 137,145, specifically incorporated by reference herein, describes camptothecin derivatives of the formula :

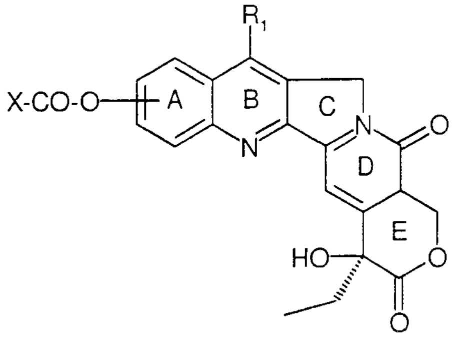

in which, in particular, $R_1$ is hydrogen, halogen or alkyl; X is a chlorine atom, or $NR_2R_3$, in which $R_2$ and $R_3$, which may be identical or different, may represent a hydrogen atom, an optionally substituted alkyl radical, a carbocycle or a heterocycle which are optionally substituted, or alkyl radicals (optionally substituted) forming, with the nitrogen atom to which they are attached, a heterocycle optionally containing another heteroatom chosen from O, S, and/or $NR_4$, wherein $R_4$ is a hydrogen atom or an alkyl radical; and in which the group X-CO-O- is located on ring A in position 9, 10, or 11.

These camptothecin derivatives are anticancer agents which inhibit topoisomerase I, among which irinotecan, in which X-CO-O- is [4-(1-piperidino-1-piperidino]carbonyloxy, is an active principle which is particularly effective in treatment of solid tumors. Camptothecin and camptothecin derivatives such as irinotecan are cytotoxic alkaloids which possesses strong anti-tumor activities. Irinotecan shows clinical activity against colon, gastric, ovarian, and small cell lung cancers, as well as non-Hodgkin's lymphoma (Bissery, M. et al., Anti Cancer Drugs, 7:166-174 (1996)).

The European patent application EP 74,256 also describes other camptothecin derivatives which are also mentioned as anticancer agents, in particular, derivatives of a structure analogous to the structure given above and in which X-CO-O- is replaced with a radical-X'R' for which X'is O or S, and R' is a hydrogen atom or an alkyl or acyl radical.

Other camptothecin derivatives have also been described, for example, in the following publications, patents, or patent applications: EP 56,692 ; EP 88,642 ; EP 296,612 ; EP 321,122 ; EP 325,247 ; EP 540,099 ; EP 737,686 ; WO 90/03169 ; WO 96/37496 ; WO 96/38146 ; WO 96/38449 ; WO 97/00876 ; U.S. 7,104,894 ; JP 57 116,015 ; JP 57 116,074 ; JP 59 005,188 ; JP 60 019,790 ; JP 01249,777 ; JP 01 246,287; and JP 91 12070 ; Canc. Res., 38 (1997) Abstr. 1526 or 95 (San Diego, April 12-16) *;* Canc. Res., 55(3):603-609 (1995) : or AFMC Int. Meci. Chem. Symp. (1997) Abstr. PB-55 (Seoul, Korea; July 27-August 1).

Camptothecin derivatives are usually administered by injection, more particularly intravenously in the form of a sterile solution or an emulsion. Camptothecin derivatives, however, can also be administered orally, in the form of solid or liquid compositions.

However, while camptothecin and camptothecin derivatives are considered to be some of the most powerful substances possessing anti-tumor activity, for example in colorectal cancers, the use of these compounds can be improved by association with other antitumor agents.

Among such antitumor agents are alkylating agents which have antineoplastic activity. Such alkylating agents include *inter alia* melphalan (alkeran, L-3-{para-[Bis(2-chloroethyl)amino]phenyl}alanine, CB 3025, phenylalanine mustard. L-Sarcolysine, SK-15673), dacarbazine (DTIC-Dome®, DTIC, dimethyl triazeno imidazole carboxamide; 5-(3,3-dimethyl-1-triazenyl)-1H-imidazole-4-carboxamide, 9CI, DIC), and cyclophosphamide (CPA, CYTOXAN®, NEOSAR®).

It has been discovered that combinations of camptothecin and an alkylating agent such as cyclophosphamide

significantly reduce the development of tumor volume compared to the development of tumor volume from the administration of each compound alone, as predicted from administration to tumor-infected mammals.

The combination of CPT-11 and cyclophosphamide has been studied in Japan (Furuta, Tomio et al., Cancer Chemotherapy, 18(3): 393-402 (1991)). In that study, however, the evaluation of the combination was only conducted on L1210 mouse leukemia, not on solid tumors. The route of administration of CPT-11 and cyclophosphamide was via the abdominal cavity, that is, the drugs were administered intraperitoneally and not orally or intravenously. Furthermore, Furuta et al. did not evaluate the effect of the highest non-toxic dose of either camptothecin or cyclophosphamide as single agents. Without such a determination, it is not possible to determine the synergistic effect of the CPT-11/cyclophosphamide combination.

It has now been found that the combination of CPT-11 and cyclophosphamide is more active at a lower dose than the highest non-toxic dose of each single agent for the treatment of cancers, including, for example, mammary adenocarcinoma.

The efficacy of a combination may be demonstrated by determination of therapeutic synergy. A combination manifests therapeutic synergy if it is therapeutically superior to one or other of the constituents used at its optimum dose (T.H. Corbett et al., Cancer Treatment Reports, 66: 1187 (1982)).

The efficacy of a combination may also been demonstrated by comparison of the maximum tolerated dose of the combination with the maximum tolerated dose of each of the separate constituents in the study in question. This efficacy may be quantified, for example by the $\log_{10}$ cell kill, which is determined by the following formula:

$$\log_{10} \text{ cell kill} = \text{T-C(days)}/3.32 \text{ x } T_d$$

in which T-C represents the time taken for the cells to grow, which is the mean time in days for the tumors of the treated group (T) to reach a predetermined value (1 g for example) and the tumors of the control group (C) to reach the same value, and $T_d$ represents the time in days needed for the volume of the tumor in the control group to double (T.H. Corbett et al., Cancer, 40: 2660-2680 (1977); F.M. Schabel et al., Cancer Drug Development, Part B, Methods in Cancer Research, 17: 3-51, New York, Academic Press Inc. (1979)). A product is considered to be active if the $\log_{10}$ cell kill is greater than or equal to 0.7. A product is considered to be very active if the $\log_{10}$ cell kill is greater than 2.8.

It has now been found that administration of CPT-11 in combination with cyclophosphamide in the following manner with the following schedules results in a combination that is very active against cancers. Furthermore, the combination of CPT-11/cyclophosphamide is more active at a lower dose than the highest non-toxic dose of either CPT-11 or cyclophosphamide alone.

The products may be administered simultaneously, semi-simultaneously, separately, or spaced out over a period of time so as to obtain the maximum efficacy of the combination. As a result, the invention is not limited to the compositions obtained by the physical association of the drugs, but also includes those which permit separate administration, either simultaneously, semi-simultaneously, or spaced out over a period of time.

## Example 1

The effect of the combination of CPT-11 and cyclophosphamide was evaluated in a three am study in mice bearing mammary adenocarcinoma MA 16/C/sp. In the first arm, four dose levels of CPT-11 were given orally on days three through seven. In the second arm, five dose levels of cyclophosphamide were given intravenously on days three, five and seven. In the combination third arm, eight dosage levels of CPT-11 were administered orally on days three through seven, with administration of eight dosage levels of cyclophosphamide intravenously on days three, five and seven. This third arm illustrated an example of semi-simultaneous administration. The results obtained in the study of single agents CPT-11 and cyclophosphamide and the combination CPT-11/cyclophosphamide are given below in Table I.

**Table I**

| Agent | Route | Dosage (mg/kg/adm) | Schedule (days) | $Log_{10}$ cell kill | T-C (days) | Time for median tumor to reach 750 mg in days | Comments |
|---|---|---|---|---|---|---|---|
| **Evaluation of CPT-11 in Combination with Cyclophosphamide (CPA) Against Mammary Adenocarcinoma MA/C/sp on C3H/HeN Female Mice CM-929** | | | | | | | |
| CPT-11 | p.o., 0.2 ml | 103.0 | 3-7 | - | - | -- | Toxic |
| CPT-11 | p.o., 0.2 ml | 64.0 | 3-7 | - | - | -- | Toxic |
| CPT-11 | p.o., 0.2 ml | 40.0 | 3-7 | 2.0 | 7.3 | 15.5 | HNTD Active |
| CPT-11 | p.o., 0.2 ml | 25.0 | 3-7 | 1.1 | 4.1 | 12.3 | Active |
| CPA | i.v., 0.2 ml | 156.0 | 3,5,7 | NTBA | NTBA | NTBA | Toxic |
| CPA | i.v., 0.2 ml | 97.0 | 3,5,7 | 2.8 | 10.3 | 18.5 | HNTD Highly Active |
| CPA | i.v., 0.2 ml | 60.0 | 3,5,7 | 1.9 | 6.9 | 15.1 | Active |
| CPA | i.v., 0.2 ml | 37.0 | 3,5,7 | 1.0 | 3.8 | 12.0 | Active |
| CPA | i.v., 0.2 ml | 23.0 | 3,5,7 | -- | -- | -- | Inactive |
| CPT-11 +<br>CPA | p.o, 0.2 ml<br>i.v., 0.2 ml | 64.0<br>60.0 | 3-7<br>3,5,7 | NTBA | NTBA | NTBA | Toxic |
| CPT-11 +<br>CPA | p.o, 0.2 ml<br>i.v., 0.2 ml | 57.6<br>54.0 | 3-7<br>3,5,7 | 3.5 | 12.9 | 21.1 | HNTD Highly Active |
| CPT- 11 +<br>CPA | p.o, 0.2 ml<br>i.v., 0.2 ml | 48.0<br>45.0 | 3-7<br>3,5,7 | 2.9 | 10.7 | 18.9 | Highly Active |
| CPT- 11 +<br>CPA | p.o, 0.2 ml<br>i.v., 0.2 ml | 38.4<br>36.0 | 3-7<br>3,5,7 | 2.8 | 10.2 | 18.4 | Highly Active |
| CPT-11 +<br>CPA | p.o, 0.2 ml<br>i.v., 0.2 ml | 28.8<br>27.0 | 3-7<br>3,5,7 | 2.0 | 7.2 | 15.4 | Active |
| CPT-11 +<br>CPA | p.o, 0.2 ml<br>i.v., 0.2 ml | 19.2<br>18.0 | 3-7<br>3,5,7 | 1.3 | 4.7 | 12.9 | Active |
| CPT-11 +<br>CPA | p.o, 0.2 ml<br>i.v., 0.2 ml | 51.2<br>24.0 | 3-7<br>3,5,7 | 2.7 | 10.0 | 18.2 | Active |
| CPT-11+<br>CPA | p.o, 0.2 ml<br>i.v., 0.2 ml | 25.6<br>48.0 | 3-7<br>3,5,7 | 2.9 | 10.7 | 18.9 | Highly Active |
| CPA: cyclophosphamide; HNTD: highest non-toxic dose; NTBA: non tumor-bearing animals; p.o.: per os; i.v.: intravenous; T-C: tumor growth delay | | | | | | | |

The combination of cyclophosphamide and irinotecan was therapeutically superior to either of the single agents used at its optimum dose. The CPT-11/cyclophosphamide combination at its highest non toxic dose produced a $log_{10}$ cell kill of 3.5, while the $log_{10}$ cell kill of the highest non toxic dose of both CPT-11 and cyclophosphamide as single

agents were 2.0 and 2.8, respectively. Therefore, it can be seen that the CPT-11 /cyclophosphamide combination was synergistically active and highly active against mammary adenocarcinoma at the highest non-toxic combination dose level, and active or highly active at other combination dose levels. The combination was therefore therapeutically superior to both of the single agents used at its optimum dose. Additionally, the combination showed greater therapeutic activity, in that the time for a median tumor to reach 750 mg in days was longer at the highest non-toxic combination dose level than in either single agent administration of irinotecan or cyclophosphamide at the highest non-toxic dose. Further, the irinotecan/cyclophosphamide combination gave a broader highly active and active dose response than the individual agents.

## Example 2

The effectiveness of irinotecan combination chemotherapy methods were tested in a dose response study in a murine tumor model. Three arms were evaluated for tolerance and efficacy. Tolerance was measured by mortality, body weight loss at nadir, host recovery time, and combination toxicity index. Efficacy end points for solid tumor models were tumor growth delay (T/C), $log_{10}$ cell kill (LCK, defined above), tumor regressions (i.e., complete remission (CR), or partial remission (PR)). For non-solid tumors, such as leukemia, efficacy was measured as the increase in life span (ILS).

Combination toxicity index (CTI) was calculated as the sum of the fraction of $LD_{10}$'s for each agent used in each combination *(*Cancer Treatment Reports, 66(5): 1187-1200 (1982)). The $LD_{10}$ for the single agent was obtained by plotting the toxicity of that agent and the dosage in mg/kg as a log probit graph. Subsequently, the CTI $LD_{10}$ was obtained by plotting as a log probit graph the observed lethality and the corresponding CTI calculated as the sum of the fraction of the $LD_{10}$ of each single agent. When the CTI equals one, only 50 % of the $LD_{10}$'s of each agent can be used in combination without additional toxicity, and when the CTI equals two, 100 % of the $LD_{10}$'s of each agent can be used in combination without additional toxicity.

The optimal total dose for oral and intravenous administration routes for irinotecan alone in various murine models is indicated in Table II.

**Table II**

| Comparison of Oral and I.V. Irinotecan Administration | | | | |
|---|---|---|---|---|
| Tumor (mice) | Route | Schedule days | Optimal Total Dose mg/kg | LCK |
| C51<br>(BALB/c) | oral<br>i.v. | 5,7,9,13,15, twice daily*<br>5,7,9,13,15, twice daily* | 845<br>615 | 2.5<br>3.0 |
| C26<br>(BALB/c) | oral<br>i.v. | 3-7 twice daily*<br>twice daily* | 558<br>228 | 0.9<br>0.7 |
| P03<br>(B6D2F1) | oral<br>i.v. | twice daily*<br>twice daily* | 900<br>346.2 | 3.4†<br>3.2 † |
| MA16/C<br>(C3H/HeN) | oral<br>i.v. | 5-9<br>5-9 | 230.5<br>130.5 | 2.7<br>2.6 |
| GOS<br>(B6D2F1) | oral<br>i.v. | 3-7, twice daily*<br>3, 5, 7 twice daily* | 900<br>346.2 | 2.1<br>2.2 |
| * The two administrations were 4 hours apart. † 1/5 tumor free survivor on day 120. | | | | |

Both methods of administration resulted in similar tolerance, as measured by body weight loss (8.5 %), nadir (7 days post last administration), and recovery (5 days post nadir, i.e., 12 days post first administration). This study showed that the efficacy in tumor bearing mice was similar for oral and i.v. irinotecan administration across all five tumor models tested in three different mice strains. The oral maximum tolerated dose for irinotecan was shown to be about 1.4 to 2.6 times the i.v. maximum tolerated dose.

Cross-resistance was measured in murine leukemia cell lines. P388/CPT is a camptothecin-resistant leukemia that was established *in vitro (*Biochem. Pharmacol., 45: 339 (1993) and maintained *in vivo* by i.p. passages in DBA2 female mice. The chemosensitivity of i.p. P388/CPT was evaluated with i.v. P388 sensitive reference drugs with different mechanisms of action. Antitumor efficacy was determined at the highest non-toxic dose as percent increase in life span (ILS), where:

$$ILS = 100 \times [(\text{median day of death (MDD) of treated mice}) - (\text{MDD control mice})] \div (\text{MDD control mice})$$

A minimal level of activity equals an ILS of greater than 26%. P388/CPT was found resistant to camptothecin s.c. and CPT-11, but both camptothecin resistant and camptothecin sensitive cell lines were very sensitive to the allcylating agent cyclophosphamide. These results show that this cell line was sensitive to alkylating agents regardless of camptothecin resistance (Vrignaud, P. et al., Proc. Amer. Assoc. Cancer Res., 35: 363, Abstract No. 2163 (1994)). Table III tabulates the results from this study.

**Table III**

| Agents \ %ILS | P388 | P388/CPT (TFS) | Comment |
|---|---|---|---|
| CPT (sc) | 82 | 0 | resistant |
| CPT-11 (i.v.) | 91 | 0 | resistant |
| Cyclophosphamide (i.v.) | 245 | 153 (1/5 TS) | sensitive |

The results for irinotecan (CPT-11) administered intravenously and simultaneously with the alkylating agent cyclophosphamide are shown in Table IV.

**Table IV**

| CPT-11 plus: | Tumor site | Schedule | % HNTD of single agents | Host recovery (days) | Therapeutic response |
|---|---|---|---|---|---|
| cyclophosphamide | MA16/C, sc | simult. | 75 | 11 | ≥ |
| HNTD represents the highest nontoxic dose. ≥: Better dose response for the combination. | | | | | |

Table V compares different application methods for the alkylating agent cyclophosphamide alone and in combination, i.e., i.v. or per os (p.o.), as indicated.

**Table V**

| Agents | Tumor site | Schedule days | HNTD Dose mg/kg | | LCK | CTI |
|---|---|---|---|---|---|---|
| CPT-11,i.v.<br>cyclophosphamide, i.v.<br>combination | MA16/C, sc | 3-7<br>3,5,7 | 200<br>-<br>288 | -<br>291<br>162 | 2.0<br>2.8<br>3.5 | <br><br>≅1.5 |
| HNTD represents the highest nontoxic dose. | | | | | | |

This study confirmed the positive results obtained in Example 1. Simultaneous administration of irinotecan with cyclophosphamide at only 75 % of the highest non-toxic dose was more effective than either agent alone in a mammary adenocarcinoma model system. Cyclophosphamide and irinotecan in combination gave a very active therapeutic profile, and were more active than either agent alone. The CPT-11/cyclophosphamide combination at its highest non toxic dose produced a $\log_{10}$ cell kill of 3.5, while the $\log_{10}$ cell kill of the highest non-toxic dose of both CPT-11 and cyclophosphamide as single agents were 2.0 and 2.8, respectively. Hence, this combination was therapeutically synergistic. The CPT-11/cyclophosphamide combination was well tolerated, with a combination toxicity index of 1.5, indicating that 75 % of the highest nontoxic does of the single agent could be combined without additional toxicity.

In conclusion, the combination of an alkylating agent, such as cyclophosphamide, with irinotecan or other camptothecin derivative, is a highly active pharmaceutical composition and represents a new method for treating cancer.

## Claims

**1.** A therapeutic pharmaceutical composition, comprising an effective amount of camptothecin, or a camptothecin

derivative, in combination with an effective amount of an alkylating agent for the treatment of solid tumors.

**2.** A therapeutic pharmaceutical composition, comprising an effective amount of CPT-11, in combination with an effective amount of an alkylating agent for the treatment of solid tumors.

**3.** The composition according to one of claims 1 or 2, wherein said alkylating agent is selected from melphalan, dacarbazine, and cyclophosphamide.

**4.** The composition according to claim 3, wherein said alkylating agent is cyclophosphamide.

**5.** The composition according to one of claims 1 or 2, wherein said solid tumor is a mammary adenocarcinoma.

**6.** The composition according to claim 3, wherein said solid tumor is a mammary adenocarcinoma.

**7.** The composition according to claim 4, wherein said solid tumor is a mammary adenocarcinoma.

**8.** A synergistic therapeutic pharmaceutical composition, comprising an effective amount at least two agents, wherein at least one agent is CPT-11, in combination with an effective amount of at least one second agent, wherein said second agent is cyclophosphamide, for the treatment of solid tumors.

**9.** The composition according to claim 8, wherein the at least two agents are administered simultaneously, semi-simultaneously, or separately.

**10.** A method of treating solid tumors, comprising administering orally an effective amount of camptothecin, or a camptothecin derivative, as a first agent, in combination with intravenous administration of an effective amount of an alkylating agent as a second agent.

**11.** The method according to claim 10, wherein the camptothecin derivative is CPT-11, and the alkylating agent is cyclophosphamide.

**12.** The method according to one of claims 10 or 11, wherein the agents are administered simultaneously, semi-simultaneously, or separately.

**13.** A method of therapeutic administration of an anti-solid tumor composition, comprising an effective amount of camptothecin, or a camptothecin derivative, in combination with an effective amount of an alkylating agent for the treatment of solid tumors, wherein said administration of the effective amounts is separate or together, and is simultaneous, semi-simultaneous, or is spaced out over a period of time.

**14.** The method according to claim 13, wherein the camptothecin derivative is CPT-11, and the alkylating agent is selected from melphalan, dacarbazine, and cyclophosphamide.

**15.** The method according to claim 14, wherein the alkylating agent is cyclophosphamide.

European Patent Office

# PARTIAL EUROPEAN SEARCH REPORT

which under Rule 45 of the European Patent Convention shall be considered, for the purposes of subsequent proceedings, as the European search report

Application Number
EP 06 11 4107

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | C.A.COGGINS E.A.: "Enhancement of irinotecan (CPT-11) activity against central nervous system tumor xenografts by alkylating agents" CANCER CHEMOTHERAPY AND PHARMACOLOGY, vol. 41, no. 6, 1998, pages 485-490, XP001064572 * page 485 * * page 486 * ----- | 1-4,8,9, 13-15 | INV. A61K31/47 A61K31/675 A61P35/00 ADD. A61K31/195 A61K31/415 |
| X | C.KOLLMANNSBERGER: "Topotecan-A novel topoisomerase I inhibitor: Pharmacology and clinical experience" ONCOLOGY (BASEL), vol. 56, no. 1, 1999, pages 1-12, XP001064576 * page 1 * * page 7, column 1 * ----- -/-- | 1,3,4,13 | |
| | | | TECHNICAL FIELDS SEARCHED (IPC) |
| | | | A61K A61P |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

4

| Place of search | Date of completion of the search | Examiner |
|---|---|---|
| Munich | 16 August 2006 | Büttner, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

# PARTIAL EUROPEAN SEARCH REPORT

Application Number
EP 06 11 4107

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | C.COGGINS E.A.: "CPT-11 plus alkylator combination therapy of central nervous system tumor xenografts" PROCEEDINGS OF THE AMERICAN ASSOCIATION FOR CANCER RESEARCH ANNUAL MEETING 1997, vol. 38, 1997, page 76, XP001064570 * page 76, column 1 * ----- | 1-4,8,9, 13-15 | |
| X | R.SUPINO E.A.: "Modulation of melphalan cytotoxic activity in human melanoma cells" ANTI-CANCER DRUGS, vol. 7, no. 5, 1996, pages 604-612, XP001064583 * page 604 * ----- | 1,3,9,13 | |
| | | | TECHNICAL FIELDS SEARCHED (IPC) |
| A | DRENGLER RONALD L ET AL: "Phase I and pharmacokinetic trial of oral irinotecan administered daily for 5 days every 3 weeks in patients with solid tumors" JOURNAL OF CLINICAL ONCOLOGY, vol. 17, no. 2, February 1999 (1999-02), pages 685-696, XP002383983 ISSN: 0732-183X * page 692, column 2, last paragraph * * page 688, column 1, paragraph 2 * ----- | 1-15 | |
| A | BISSERY M C ET AL: "EXPERIMENTAL ANTITUMOR ACTIVITY AND PHARMACOKINETICS OF THE CAMPTOTHECIN ANALOG IRINOTECAN (CTP-11) IN MICE" ANTI-CANCER DRUGS, RAPID COMMUNICATIONS, OXFORD, GB, vol. 7, no. 4, 1996, pages 437-460, XP008033426 ISSN: 0959-4973 * page 453, column 1, paragraph 1 * ----- | 1-15 | |
| | -/-- | | |

4

EPO FORM 1503 03.82 (P04C10)

European Patent Office

# PARTIAL EUROPEAN SEARCH REPORT

Application Number
EP 06 11 4107

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | CHOI SEUNG-HOON ET AL: "Oral versus intraperitoneal administration of irinotecan in the treatment of human neuroblastoma in nude mice" CANCER LETTERS, vol. 124, no. 1, 13 February 1998 (1998-02-13), pages 15-21, XP002383984 ISSN: 0304-3835 * page 19, column 1, last paragraph - column 2, paragraph 1 * ----- | 1-15 | |
| A | THOMPSON J ET AL: "EFFICACY OF ORAL IRINOTECAN AGAINST NEUROBLASTOMA XENOGRAFTS" ANTI-CANCER DRUGS, RAPID COMMUNICATIONS, OXFORD, GB, vol. 8, no. 4, 1997, pages 313-322, XP009058550 ISSN: 0959-4973 * page 318, column 2, paragraph 1 * ----- | 1-15 | TECHNICAL FIELDS SEARCHED (IPC) |
| X | MURREN J R ET AL: "DOSE-ESCALATION AND PHARMACODYNAMIC STUDY OF TOPOTECAN IN COMBINATION WITH CYCLOPHOSPHAMIDE IN PATIENTS WITH REFRACTORY CANCER" JOURNAL OF CLINICAL ONCOLOGY, GRUNE AND STRATTON, NEW YORK, NY, US, vol. 15, no. 1, January 1997 (1997-01), pages 148-157, XP001064587 ISSN: 0732-183X * page 149, column 1, paragraph 2 - column 2, paragraph 1 * * page 151, column 1, paragraph 2 * ----- | 1-15 | |
| | -/-- | | |

4

EPO FORM 1503 03.82 (P04C10)

European Patent Office

# PARTIAL EUROPEAN SEARCH REPORT

Application Number
EP 06 11 4107

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | GIOVANELLA B ET AL: "TREATMENT OF HUMAN CANCER XENOGRAFTS WITH CAMPTOTHECIN ANALOGUES IN COMBINATION WITH CYTOXAN OR X-IRRADIATION" PROCEEDINGS OF THE ANNUAL MEETING OF THE AMERICAN ASSOCIATION FOR CANCER RESEARCH, NEW YORK, NY, US, vol. 35, March 1994 (1994-03), page 454,AN2711, XP001064595 ISSN: 0197-016X * the whole document * ----- | 1-15 | |
| E | WO 01/28542 A (SUPERGEN, INC; RUBINFELD, JOSEPH) 26 April 2001 (2001-04-26) * page 6, line 16 * * page 9, line 29 * ----- | 1-15 | TECHNICAL FIELDS SEARCHED (IPC) |

4

EPO FORM 1503 03.82 (P04C10)

European Patent Office

INCOMPLETE SEARCH SHEET C

Application Number
EP 06 11 4107

Although claims 10-15 are directed to a method of treatment of the human/animal body (Article 52(4) EPC), the search has been carried out and based on the alleged effects of the compound/composition.

-----

**ANNEX TO THE EUROPEAN SEARCH REPORT ON EUROPEAN PATENT APPLICATION NO.** EP 06 11 4107

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2006

| Patent document cited in search report | Publication date | Patent family member(s) | Publication date |
|---|---|---|---|
| WO 0128542 A | 26-04-2001 | AU 7377200 A | 30-04-2001 |
| | | EP 1223934 A2 | 24-07-2002 |
| | | US 6191119 B1 | 20-02-2001 |
| | | US 6420378 B1 | 16-07-2002 |
| | | US 6664233 B1 | 16-12-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 19000700 P
- EP 137145 A
- EP 74256 A
- EP 56692 A
- EP 88642 A
- EP 296612 A
- EP 321122 A
- EP 325247 A
- EP 540099 A
- EP 737686 A
- WO 9003169 A
- WO 9637496 A
- WO 9638146 A
- WO 9638449 A
- WO 9700876 A
- US 7104894 B
- JP 57116015 A
- JP 57116074 A
- JP 59005188 A
- JP 60019790 A
- JP 01249777 A
- JP 1246287 A
- JP 3012070 A

### Non-patent literature cited in the description

- **BISSERY, M. et al.** *Anti Cancer Drugs,* 1996, vol. 7, 166-174
- *Canc. Res.,* 12 April 1997, vol. 38
- *Canc. Res.,* 1995, vol. 55 (3), 603-609
- *AFMC Int. Meci. Chem. Symp.,* 27 July 1997
- **FURUTA ; TOMIO et al.** *Cancer Chemotherapy,* 1991, vol. 18 (3), 393-402
- **T.H. CORBETT et al.** *Cancer Treatment Reports,* 1982, vol. 66, 1187
- **T.H. CORBETT et al.** *Cancer,* 1977, vol. 40, 2660-2680
- **F.M. SCHABEL et al.** Cancer Drug Development, Part B, Methods in Cancer Research. Academic Press Inc, 1979, vol. 17, 3-51
- *Cancer Treatment Reports,* 1982, vol. 66 (5), 1187-1200
- *Biochem. Pharmacol.,* 1993, vol. 45, 339
- **VRIGNAUD, P. et al.** *Proc. Amer. Assoc. Cancer Res.,* 1994, vol. 35, 363